(19) Europäisches Patentamt
European Patent Office
Office européen des brevets

(11) **EP 0 863 739 B1**

(12) **EUROPÄISCHE PATENTSCHRIFT**

(45) Veröffentlichungstag und Bekanntmachung des Hinweises auf die Patenterteilung:
**17.04.2002 Patentblatt 2002/16**

(21) Anmeldenummer: **97930505.9**

(22) Anmeldetag: **05.07.1997**

(51) Int Cl.$^7$: **A61K 7/00**

(86) Internationale Anmeldenummer:
**PCT/EP97/03554**

(87) Internationale Veröffentlichungsnummer:
**WO 98/11863 (26.03.1998 Gazette 1998/12)**

(54) **MITTEL ZUM FÄRBEN VON HAAREN**

HAIR DYE

COLORANT CAPILLAIRE

(84) Benannte Vertragsstaaten:
**DE ES FR GB IT**

(30) Priorität: **18.09.1996 DE 19637966**

(43) Veröffentlichungstag der Anmeldung:
**16.09.1998 Patentblatt 1998/38**

(73) Patentinhaber: **Wella Aktiengesellschaft 64274 Darmstadt (DE)**

(72) Erfinder:
• **GAST-HADULLA, Anette**
  **D-64331 Weiterstadt (DE)**
• **BRAUN, Petra**
  **D-64839 Münster (DE)**
• **BALZER, Wolfgang, R.**
  **D-64665 Alsbach (DE)**
• **DEUTZ, Herbert**
  **D-67823 Gro -Umstadt (DE)**

(56) Entgegenhaltungen:
EP-A- 0 367 926      EP-A- 0 471 606
EP-A- 0 630 643      EP-A- 0 776 652
WO-A-87/04616        WO-A-93/19722
US-A- 5 154 916      US-A- 5 496 543

• CHEMICAL ABSTRACTS, vol. 94, no. 4, 26.Januar 1981 Columbus, Ohio, US; abstract no. 20238, LISBRAN K. K., JAPAN: "Hair dyes" XP002053431 & JP 55 120 508 A (LISBRAN K. K., JAPAN)

## Beschreibung

Gegenstand der Erfindung sind Mittel zum oxidativen Färben von Haaren, insbesondere menschlichen Haaren, welche eine Kombination aus Bienenwachs und Proteinhydrolysaten und/oder Aminosäuren enthalten.

Für das Färben von Haaren kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Mit Oxidationsfarbstoffen lassen sich in der Regel intensive Färbungen mit guten Echtheitseigenschaften erzielen, die Entwicklung der Farbe geschieht jedoch unter dem Einfluß von starken Oxidationsmitteln, wie zum Beispiel Wasserstoffperoxid, was häufig Schädigungen der Faser zur Folge hat. Direktziehende Farbstoffe werden zwar unter schonenderen Bedingungen appliziert, jedoch sind die Färbeergebnisse, insbesondere bezüglich des Farbausgleiches von geschädigtem und ungeschädigtem Haar häufig nicht zufriedenstellend.

Es bestand daher die Aufgabe, ein Haarfärbemittel zur Verfügung zu stellen, das leicht anwendbar ist, eine gute Pflege und möglichst geringe Schädigung der Haare ermöglicht sowie unabhängig von der unterschiedlichen Struktur der Haare eine gleichmäßige Färbung bewirkt.

Hierzu wurde nunmehr gefunden, daß durch ein Mittel zum Färben von Haaren, welches eine Kombination von Bienenwachs und Proteinhydrolysat und/oder Aminosäuren enthält, die gestellte Aufgabe in hervorragender Weise gelöst wird.

Aus der US-PS 5 496 543 sind nicht-oxidative kosmetische Zubereitungen (z.B. Mascara) bekannt, welche Bienenwachs, Keratinhydrolysat sowie bestimmte Pigmente enthalten.

Gegenstand der vorliegenden Erfindung ist ein Mittel zum Färben von Haaren auf der Basis einer Entwicklersubstanz-Kupplersubstanz-Kombination und gegebenenfalls direktziehenden Farbstoffen, welches vor der Anwendung mit einem Oxidationsmittel vermischt wird und
dadurch gekennzeichnet ist, daß es eine Kombination aus

(a) Bienenwachs und (b) mindestens einem Proteinhydrolysat und/oder mindestens einer Aminosäure enthält.

Das Bienenwachs wird in dem erfindungsgemäßen Mittel in einer Menge von 0,1 bis 10 Gewichtsprozent, vorzugsweise 0,5 bis 5 Gewichtsprozent, eingesetzt.

Als Proteinhydrolysat werden vorzugsweise Seidenproteinhydrolysate, Keratinhydrolysate sowie deren Mischungen eingesetzt, während als Aminosäure sowohl neutrale als auch saure oder basische Aminosäuren, beispielsweise Glycin, Alanin, Valin, Leucin, Glutaminsäure und Arginin, eingesetzt werden können.

Die Menge an Proteinhydrolysat und/oder Aminosäure beträgt in dem erfindungsgemäßen Mittel vorzugsweise 0,1 bis 10 Gewichtsprozent, insbesondere 0,5 bis 5 Gewichtsprozent, wobei die Verwendung einer Kombination aus einem oder mehreren Proteinhydrolysaten mit einer oder mehreren Aminosäuren besonders bevorzugt ist.

Als direktziehende Farbstoffe können übliche, physiologisch unbedenkliche, direktziehende Farbstoffe aus der Gruppe der Nitrofarbstoffe, Azofarbstoffe, Chinonfarbstoffe und Triphenylmethanfarbstoffe wie zum Beispiel 4,N-Ethyl,N-(2'-hydroxyethyl)amino-1-(2''hydroxyethyl)amino-2-nitro-benzol,   1-Amino-3-methyl-4-(2'-hydroxyethyl) amino-6-nitro-benzol, 2,2'-[(Amino-3-nitrophenyl)imino]-bisethanol Hydrochlorid (HC Red 13), 1-(2'-Hydroxyethyl)amino-2-nitro-4-bis-(2''-hydroxyethyl)amino-benzol, 4-Bis-(2'-Hydroxyethyl)amino-1-(2''-methoxyethyl)amino-nitrobenzol, 1-(2',3'-Dihydroxypropyl)amino-2-nitro-4-[methyl-(2''-hydroxyethyl)amino]-benzol,   1-[(2',3'-Dihydroxypropyl)amino]-2-nitro-4-[ethyl-2''-(hydroxyethyl)amino]-benzol,   1-(3'-Hydroxypropylamino)-2-nitro-4-bis-(2''-hydroxyethylamino)-benzol,   1-Amino-4-(2'-hydroxyethyl)-amino-nitrobenzol,   1-Hydroxy-2-amino-4,6-dinitro-benzol,   1-Amino-2-nitro-4-bis-(2'-hydroxyethyl)amino-benzol,   1-Amino-2-nitro-4-(2'-hydroxyethyl)amino-5-chlorbenzol,   1-(2'-Hydroxyethyl) amino-2-nitro-4-amino-benzol, 1-Hydroxy-3-nitro-4-amino-benzol, 1-(2'-Aminoethyl)amino-2-nitro-4-(2''hydroxyethyl)-oxybenzol,   3-Nitro-4-(2'-hydroxyethyl)-amino-phenylglycerinether,   1-Amino-5-chlor-4-(2',3,dihydroxypropyl)amino-2-nitro-benzol,   1,4-Bis-[(2',3'dihydroxypropyl)amino]-5-chlor-2-nitro-benzol,   1-Hydroxy-2-(2'hydroxyethyl)amino-4,6-dinitro-benzol,   2-Amino-6-chlor-4-nitrophenol,   1-Hydroxy-3-nitro-4-(3'hydroxypropylamino)-benzol,   3-Nitro-4-ethylaminobenzoesäure,   4-Amino-2-nitro-diphenylamino-2-carbonsäure,   2-Chlor-6-ethylamino-4-nitrophenol, 2,5-Diamino-6-nitropyridin, 1,2,3,4-Tetrahydro-6-nitrochinoxalin, 4-(2'-Hydroxyethyl)amino-3-nitro-benzonitril, 4-(2'-Hydroxyethyl)amino-3-nitro-benzamid, 1-Amino-2-(2'hydroxyethyl)amino-5-nitro-benzol, 1-Methoxy-2-(2'hydroxyethyl)amino-5-nitro-benzol,   1-Hydroxy-3-nitro-4-(2'-hydroxyethyl)amino-benzol,   1-Hydroxy-2-amino-3-nitro-benzol, 1-Amino-2-methyl-6-nitro-benzol, 1-(2'Hydroxyethyl)-oxy-3-methylamino-4-nitro-benzol, 1-Methylamino-2-nitro-5-(2',3'-dihydroxypropyl)-oxybenzol, 1-(2'-Hydroxyethyl)amino-2-hydroxy-4-nitrobenzol, 1-Methoxy-3-(2'-aminoethyl)-amino-4-nitrobenzol,   1-(2'-Ureidoethyl)amino-4-nitro-benzol,   1-(2'-Hydroxyethyl)amino-2-nitro-benzol,   4-(2'-Hydroxyethyl)amino-3-nitro-trifluormethyl-benzol, 2,4-Bis-[N-(2'-hydroxyethyl)amino]-5-chlor-nitrobenzol, 4-(2',3'-Dihydroxypropyl)amino-3-nitro-trifluormethyl-benzol,   4-(2'-Hydroxymethyl)amino-3-nitro-methylbenzol, 4-(2'-Hydroxyethyl)amino-3-nitro-chlorbenzol,   1-(4'-Nitrophenylazo)-2-methyl-4-bis-(2''-hydroxyethyl)aminobenzol,   1-(3'-Nitro-4-amino)-phenylazo-2-hydroxy-7-trimethyl-ammoniumchlorid-naphthalin, 1-(2'Hydroxy-4' sulfo-6'nitro)-naphthylazo-2-hydroxynaphthalin, 1-(4'-Aminophenylazo)-2-methyl-4-bis-[(2'-hydroxyethyl)-amino]-benzol, 5-(4'-Dimethylaminophenylazo)-1,4-dimethyl-triazoniumchlorid, 1-(2'-Methoxyphenylazo)-2-hydroxy-7-trimethylammonium-naphthalinchorid, 1-(4'-Aminophenylazo)-2-hydroxy-7-trimethylammoniumnaphthalin, 4-(3'-Trimethylammoniumphenylazo)-N-phenyl-3-methyl-pyrazolon(5), 4-Hydroxy-3-[(4'-sulfo-1'naphthyl)azo]-1-naphthalinsulfonsäure, 1-(4'-Sulfophenylazo)-2-hydroxynaphthalin, 1-(4'-Sulfonphenylazo)-2-hydroxy-6-sulfo-naphthalin, 4-Amino-[4'-bis-(2"hydroxyethyl)amino]-azobenzol, 4-Amino-[4'-bis-(2"hydroxyethyl)amino]-2'-methyl-azobenzol, 3-(2',6'-Diaminopyridyl-3'-azo)-pyridin, 7-Phenylazo-1-amino-3,6-disulfo-8-hydroxy-naphthalin, 5-Acetylamino-4-hydroxy-3-[(2'-methylphenyl)azo]-2,7-naphthalindisulfonsäure, 2-(2',4'-Dimethylphenylazo)-6-(4"-sulfophenylazo)-1,3-dihydroxybenzol, 1,4-Bis-(2',3'-dihydroxypropyl) amino-anthrachinon, 1-Methylamino-4-(2'hydroxyethyl)amino-anthrachinon, 2-(2'-Aminoethyl)-amino-anthrachinon, 2-Brom-4,8-diamino-6-(3'-trimethylammonium)-phenylamino-1,5-naphthochinon, 1-(2'-Sulfo-4'-methyl-phenyl)-amino-4-hydroxy-anthrachinon, 1,4-Diamino-anthrachinon, 1-Amino-2-sulfo-4-cyclohexylamino-anthrachinon, 1-Methylamino-4-aminopropylamino-anthrachinon, 1-Aminopropylamino-anthrachinon, 4',4",4"'-Triamino-3-methyl-triphenylcarboniumchlorid, Bis-(4,4-Diethylaminophenyl)-4'-ethylamino-naphthylcarboniumchlorid, Bis-(4,4-Dimethylaminophen)-4'phenylamino-naphthyl-carbonsiumchlorid und 4,4-Bis-(N-Ethyl-3-sulfobenzyl)-amino-2"-sulfofuchsonium, 2,4-Dinitro-1-naphthol-7-sulfonsäure-Dinatriumsalz (Acid Yellow 1; CI 10 316);2-(2'-Chinolyl)-1H-indene-1,3(2H)-dion-monodisulfon-säure-Dinatriumsalz (Acid Yellow 3; CI 47 005); 4,5-Dihydro-5-oxo-1-(4'-sulfophenyl)-4-[(4"-sulfo-phenyl) azo]-1H-pyrazol-3-carbonsäure-Trinatriumsalz (Acid Yellow 23; CI 19 140); 3',6'-Dihydroxyspiro [isobenzofuran-1 (3H), 9'(9H)-xanthen]-3-on (Acid Yellow 73; CI 45 350:1); 5-[2',4'-Dinitrophenyl)amino]-2-(phenylamino)-benzol-sulfonsäure-Natriumsalz (Acid Orange 3; CI 10 385); 4-[(2',4'-Dihydroxyphenyl)azo]-benzolsulfonsäure-Natriumsalz (Acid Orange 6; CI 14 270); 4-[2'-Hydroxy-1'-naphthyl)-azo]-benzolsulfonsäure-Natriumsalz (Acid Orange 7; CI 15 510); 4-[[3'-[(2",4"-Dimethylphenyl) azo]-2',4'-dihydroxyphenyl]azo]-benzolsulfonsäure-Natriumsalz (Acid Orange 24;CI 20 170); 4-Hydroxy-3-[(4'-sulfo-1'-naphthyl)azo]-1-naphthalinsulfonsäure-Dinatriumsalz (Acid Red 14; CI 14 720); 7-Hydroxy-8-[(4'-sulfo-1'-naphthyl)azo]-1,3-naphthalindisulfonsäure-Trinatriumsalz (Acid Red 18; CI 16 255); 3-Hydroxy-4-[(4'-sulfo-1'-naphthyl)azo]-2,7-naphthalindisulfonsäure-Trinatriumsalz (Acid Red 27; CI 16 185); 5-Amino-4-hydroxy-3-phenylazo-2,7-naphtalindisulfonsäure-Dinatriumsalz (Acid Red 33; CI 17 200); 5- (Acetylamino) -4-hydroxy-3-[(2'-methylphenyl)azo]-2, 7-naphthalindisulfonsäure-Dinatriumsalz (Acid Red 35; CI 18 065); 3',6'-Dihydroxy-2',4',5',7'-tetraiodospiro[isobenzo-furan-1(3H), 9' (9H) -xanthen]-3-on-Dinatriumsalz (Acid Red 51; CI 45 430); 3,6-Bis-(diethylamino)-9-(2',4'-disulfophenyl)-xanthyliumhydroxid-Natriumslz (Acid Red 52; CI 45 100); 7-Hydroxy-8-[[4'-(phenylazo) phenyl]azo]-1,3-naphthalindisulfonsäure-Dinatriumsalz (Acid Red 73; CI 27 290); 2',4',5',7'-Tetrabromo-3',6'dihydroxyspiro [isobenzo-furan-1(3H),9'(9H)-xanthen]-3-on-Dinatriumsalz (Acid Red 87; CI 45 380);2',4',5',7'-Tetrabromo-4,5,6,7-tetrachloro-3',6'-dihydroxyspiro[isobenzofuran-1 (3H), 9'(9H)-xanthen]-3-on-Dinatriumsalz (Acid Red 92; CI 45 410); 3', 6'-Dihydroxy-4',5'-dijodospiro[isobenzofuran-1(3H),9'(9H)-xanthen]-3-on-Dinatriumsalz (Acid Red 95; CI 45 425); Acid Red 195; Acid Blue 9 (CI 42 090); 2,2'-[(9,10-Dihydro-9,10-dioxo-1,4-anthracendiyl)-diimino]-bis-(5-methyl-benzolsulfonsäue)-Dinatriumsalz (Acid Green 25; CI 61 570); N-[4-[[4'-(Dimethylamino)phenyl]-(2"-hydroxy-3",6"disulfo-1"-naphthyl)-methylen]-2,5-cyclohexadien-1-yliden]-N-methylmethanaminiumhydroxid (Acid Green 50; CI 44 090); N-[4-[[4'-Diethylamino)phenyl]-(2",4"disulfophenyl)-methylen]-2,5-cyclohexadien-1-yliden]-N-ethylethan- aminiumhydroxid-Natriumsalz (Acid Blue 1; CI 42 045); N-[4-[[4'-Diethylamino)phenyl]- (5"-hydroxy-2",4"-disulfo-phenyl)-methylen]-2,5-caclohexadien-1-yliden]-N-ethyl-ethanaminiumhydroxid-Calciumsalz (Acid Blue 3;CI 42 051);

1-Amino-4-(cyclohexylamino)-9,10-dihydro-9,10-dioxo-2-anthracensulfonsäure-Natriumsalz (Acid Blue 62; CI 62 045) ;

2-(1',3'-Dihydro-3'-oxo-5'-sulfo-2'H-indol-2'-yliden)-2,3-dihydro-3-oxo-lH-indol-5-sulfonsäure-Dinatriumsalz (Acid Blue 74; CI 73 015);9-(2'-Carboxyphenyl)-3-[(2"methylphenyl)amino]-6-[(2"'-methyl-4"'-sulfophenyl)-amino)]-xanthylium-hydroxid-Natriumsalz (Acid Violet 9; CI 45 190);

2-[(9',10'-Dihydro-4'-hydroxy-9',10'-dioxo-1'anthracenyl)-amino]-5-methylbenzolsulfonsäure-Natriumsalz (Acid Violet 43; CI 60 730);

3,3'-[Sulfonyl-bis(2-nitro-4,1-phenylen)imino]-bis-[6-phenylamino)-benzol-dinatriumsulfonat] (Acid Brown 13; CI 10 410);

4-Amino-5-hydroxy-3-[(4'-nitrophenyl)azo]-6-(phenylazo)-2,7-naphthalindisulfonsäure-Dinatriumsalz (Acid Black 1; CI 20 470); 3-Hydroxy-4-[(2'-hydroxy-1'-naphthyl)-azo]-7-nitro-1-naphthalinsulfonsäure-Natriumsalz (Acid Black 52; CI 15 711);

N-[4-[[4'-(Diethylamino)phenyl]-[4"-(ethylamino)-1"naphthyl]-methylen]-2,5-cyclohexadien-1-yliden]-N-ethylethanammoniumchlorid (Basic Blue 7; CI 42 595); 4-[(4'-Aminophenyl)-(4'-imino-2',5'-cyclohexadien-1'yliden)-methyl]-2-methyl-aminobenzol-Hydrochlorid (Basic Violet 14; CI 42 510); 4-(Acetylamino)-5-hydroxy-6-[[7' -sulfo-4' -[(4"-Sulfophenyl)azo]-1'naphthyl]azo]-1,7-naphthalin-disulfonsäure-Tetranatriumsalz (Brilliant Black 1; CI 28 440);

[8-(p-Aminophenyl)azo]-7-hydroxy-2-naphthyl]-trimethylammoniumchlorid (Basic Brown 16; CI 12 250);

[8-[4' -Amino-2' -nitrophenyl) azo]-7-hydroxy-2-naphthyl]-trimethylammoniumchlorid (Basic Brown 17; CI 12 251); 7-Hydroxy-8-[(2'-methoxyphenyl)azo]-N,N,N-trimethyl-2-naphthylammoniumchlorid (Basic Red 76; CI 12 245); 3-[(4'-Amino-6'-bromo-5',8'-dihydro-1'-hydroxy-8'imino-5'-oxo-2'-naphthyl)amino]-N,N,N-trimethylammoniumchlorid (Basic Blue 99; CI 56 059) verwendet werden.

Vorzugsweise werden die folgenden Entwicklersubstanzen und Kupplersubstanzen eingesetzt:

**(A) Entwicklersubstanzen:**

2,5-Diaminotoluol, 2,5-Diaminophenylethanol, p-Aminophenol, 1,4-Diaminobenzol, 1-Methyl-2,5-diaminobenzol, 1-Amino-4-bis(2'-hydroxyethyl)-amino-benzol, 2,4,5,6-Tetraaminopyrimidin, 1-Hydroxy-3-methyl-4-amino-benzol, 1-(2'-Hydroxyethyl)-2, 5-diaminobenzol, 4-Amino-2-aminomethylphenol, 4-Amino-1-hydroxy-2-(2'-hydroxyethyl)-aminomethylphenol, 4-Hydroxy-2,5,6-triaminopyrimidin, 4,5-Diamino-1-(4'-chlorbenzyl)-pyrazol-Hemisulfat und 4-Amino-m-cresol.

**(B) Kupplersubstanzen:**

Resorcin, 4-Chlorresorcin, 4,6-Dichlorresorcin, 2-Methylresorcin, 2,4-Diamino-5-fluor-toluol, 2-Amino-4-(2'-hydroxyethyl)amino-anisol, 1,3-Diaminobenzol, 5-Amino-2-methylphenol, 2,4-Diaminophenoxyethanol, 1-Naphthol, m-Aminophenol, 3-Amino-4-chlor-6-methylphenol, 3-Amino-2-chlor-6-methylphenol, 3-Amino-4-fluor-6-methylphenol, 3-Amino-4-methoxy-6-methylphenol, 3-Amino-2-methylphenol, 4-Amino-2-hydroxy-phenoxyethanol, 4-Hydroxy-1,2-methylendioxybenzol, 4-(2'-Hydroxyethyl)amino-1,2-methylendioxybenzol, 2,4-Diamino-5-ethoxytoluol, 4-Hydroxyindol, 3,5-Diamino-2,6-dimethoxypyridin, 1-Methyl-2-hydroxy-4-(2'hydroxyethyl)amino-benzol, 1,3-Bis-(2',4'-diamino-phenoxy)-propan, 2,4-Diamino-5-methyl-phenetol, 2,4-Diamino-5-(2'-hydroxyethoxy)-toluol, 3-Amino-2-methylamino-6-methoxy-pyridin, 5-Amino-4-fluor-2-methylphenol-Hemisulfat und 2,4-Diamino-5-fluor-toluol-Sulfathydrat.

Das erfindungsgemäße Mittel enthält einen oder mehrere der vorgenannten Entwicklersubstanzen und Kupplersubstanzen sowie gegebenenfalls einen oder mehrere der vorgenannten direktziehenden Farbstoffe, wobei die Farbstoffe sofern es Basen sind, auch in Form der physiologisch verträglichen Säureadditionssalze, beispielsweise als Hydrochlorid beziehungsweise Sulfat, oder - sofern sie aromatische OH-Gruppen besitzen - in Form der Salze mit Basen, zum Beispiel als Alkaliphenolat, eingesetzt werden können.

Falls direktziehende Farbstoffe in dem erfindungsgemäßen Mittel enthalten sind, werden sie in einer Menge von etwa 0,01 bis 5 Gewichtsprozent, vorzugsweise 0,05 bis 3 Gewichtsprozent, eingesetzt.

Die Einsatzmenge der Entwicklersubstanzen und Kupplersubstanzen beträgt jeweils etwa 0,01 bis 5 Gewichtsprozent, vorzugsweise 0,1 bis 3 Gewichtsprozent, wobei die Gesamteinsatzmenge der Entwicklersubstanzen und Kupplersubstanzen 0,02 bis 10 Gewichtsprozent, insbesondere 0,2 bis 6 Gewichtsprozent, beträgt.

Die Entwicklersubstanzen und Kupplersubstanzen werden im allgemeinen in etwa äquimolaren Mengen eingesetzt. Es ist jedoch nicht nachteilig, wenn die Entwicklersubstanz in einem gewissen Überschuß oder Unterschuß vorhanden ist. Weiterhin kann das Haarfärbemittel dieser Anmeldung zusätzlich andere Farbkomponenten, beispielsweise 6-Amino-2-methylphenol, 3-(2,6-Diaminopyridyl-3-azo)-pyridin und 2-Amino-5-methylphenol, enthalten.

Das erfindungsgemäße Haarfärbemittel liegt vorzugsweise in Form einer Emulsion, eines Geles oder insbesondere einer Creme vor.

Der pH-Wert des erfindungsgemäßen Haarfärbemittels liegt im Bereich von 7,5 bis 12, vorzugsweise 9 bis 11, wobei der pH-Wert des gebrauchsfertigen Oxidationshaarfärbemittels (das heißt der Mischung des erfindungsgemäßen Haarfärbemittels mit dem Oxidationsmittel) gleich 5,5 bis 10, vorzugsweise 6 bis 9, ist.

Je nach Zusammensetzung und gewünschtem pH-Wert des Haarfärbemittels erfolgt die Einstellung des pH-Wertes vorzugsweise mit Ammoniak oder organischen Aminen, wie zum Beispiel Glucaminen, Aminomethylpropanol, Monoethanolamin oder Triethanolamin, anorganischen Basen, beispielsweise Natriumhydroxid, Kaliumhydroxid, Natriumcarbonat oder Calciumhydroxid, beziehungsweise organischen oder anorganischen Säuren, wie zum Beispiel Milchsäure, Zitronensäure, Essigsäure oder Phosphorsäure.

Selbstverständlich kann das vorstehend beschriebene Haarfärbemittel gegebenenfalls weitere, für Haarfärbemittel übliche Zusätze, wie zum Beispiel Konservierungsstoffe und Parfümöle; zur Einstellung der Viskosität Salze, beispielsweise Natriumchlorid oder Natriumsulfat; Antioxidantien, beispielsweise Natriumsulfit, Thioglykolsäure oder Ascorbinsäure; Komplexbildner; Lösungsmittel wie Wasser, niedere aliphatische Alkohole, beispielsweise Ethanol, Propanol und Isopropanol, oder Glykole wie Glycerin und 1,2-Propylenglykol; Netzmittel oder Emulgatoren aus den Klassen der anionischen, kationischen, amphoteren oder nichtionogenen oberflächenaktiven Substanzen; Verdicker wie höhere Fettalkohole, Stärke oder Cellulosederivate; weiterhin Weichmacher; Vaseline; Paraffinöl und Fettsäuren sowie außerdem Pflegestoffe, wie kationische Harze, Lanolinderivate, Cholesterin, Vitamine, Pantothensäure und Betain, enthalten. Die erwähnten Bestandteile werden in den für solche Zwecke üblichen Mengen verwendet, zum Beispiel die Netzmittel und Emulgatoren in Konzentrationen von 0,1 bis 30 Gewichtsprozent, die Verdicker in einer Menge von 0,1 bis 25 Gewichtsprozent und die Pflegestoffe in einer Konzentration von 0,1 bis 5,0 Gewichtsprozent.

Besonders vorteilhaft ist hierbei der Zusatz von nicht-ionischen und/oder anionischen Netzmitteln oder Emulgatoren, wie zum Beispiel Fettalkoholsulfaten, insbesondere Laurylsulfat und Natriumcocoylsulfat, oxethylierten Fettalkoholsulfaten, insbesondere Natriumlaurylethersulfaten mit 2 bis 4 Ethylenoxideinheiten im Molekül, oxethylierten Fettsäureestem, oxethylierten Nonylphenolen, oxethylierten Fettalkoholen, Alkylbenzolsulfonaten oder Fettsäurealkanolamiden, in einer Gesamtmenge von etwa 0,1 bis 30 Gewichtsprozent, vorzugsweise 0,2 bis 15 Gewichtsprozent, zu dem erfindungsgemäßen Haarfärbemittel.

Für die Anwendung zur oxidativen Haarfärbung vermischt man das vorstehend beschriebene Haarfärbemittel unmittelbar vor dem Gebrauch mit einem Oxidationsmittel und trägt eine für die Haarfärbung ausreichende Menge, je nach Haarfülle im allgemeinen etwa 60 bis 200 Gramm, der gebrauchsfertigen Zubereitung auf das Haar auf.

Als Oxidationsmittel zur Entwicklung der Haarfärbung kommen hauptsächlich Wasserstoffperoxid oder dessen Additionsverbindungen an Harnstoff, Melamin oder Natriumborat in Form einer 1- bis 12prozentigen, vorzugsweise 1,5- bis 6prozentigen wäßrigen Lösung in Betracht. Das Mischungsverhältnis von Haarfärbemittel zu Oxidationsmittel ist abhängig von der Konzentration des Oxidationsmittels und beträgt in der Regel 5:1 bis 1:5, vorzugsweise 1:1, wobei der Gehalt an Oxidationsmittel in der gebrauchsfertigen Zubereitung etwa 0,5 bis 8 Gewichtsprozent, insbesondere 1 bis 4 Gewichtsprozent, betragen sollte.

Man läßt das Gemisch bei 15 bis 50 Grad Celsius etwa 10 bis 45 Minuten, vorzugsweise 20 Minuten lang bei 40 Grad Celsius, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Weinsäure, nachgespült. Anschließend wird das Haar getrocknet.

Das erfindungsgemäße Haarfärbemittel ermöglicht eine intensive, schonende und pflegende Färbung der Haare, wobei aufgrund des besseren Farbausgleiches eine gleichmäßige Färbung der Haare von dem ungeschädigten Haaransatz bis zu den stark geschädigten Haarspitzen erzielt werden kann.

Die nachfolgenden Beispiele sollen den Gegenstand der Erfindung näher erläutern ohne diesen hierauf zu beschränken.

**Beispiele**

| Beispiel 1: | Oxidationshaarfärbemittel |
|---|---|
| 15,00 g | Cetylalkohol |
| 3,50 g | Natriumlaurylalkohol-diglykolether-sulfat (28prozentige wäßrige Lösung) |
| 3,00 g | Monoethanolamin |
| 1,30 g | 2,5-Diaminotoluol |
| 1,00 g | Bienenwachs |
| 1,00 g | Keratinhydrolysat |
| 0,65 g | Resorcin |
| 0,30 g | Ascorbinsäure |
| 74,25 g | Wasser |
| 100,00 g | |

50 g des vorstehenden Haarfärbemittels werden unmittelbar vor Gebrauch mit 50 g einer 2prozentigen wäßrigen Wasserstoffperoxidlösung vermischt. Das erhaltene Gemisch wird anschließend auf blonde Naturhaare aufgetragen. Nach einer Einwirkungszeit von 30 Minuten bei 40 Grad Celsius wird das Haar mit Wasser gespült und getrocknet.

Das Haar besitzt einen gleichmäßigen mittelblonden Naturton und ist deutlich gepflegter als bei Verwendung des gleichen Oxidationshaarfärbemittels, welches jedoch kein Bienenwachs und kein Proteinhydrolysat enthält.

| Beispiel 2: | Oxidationshaarfärbemittel |
|---|---|
| 15,00 g | Cetylalkohol |
| 3,50 g | Natriumlaurylalkohol-diglykolethersulfat (28prozentige wäßrige Lösung) |
| 3,00 g | Monoethanolamin |
| 1,30 g | 1-Methyl-2,5-diaminobenzol |
| 1,00 g | Bienenwachs |

(fortgesetzt)

| **Beispiel 2:** | Oxidationshaarfärbemittel |
|---|---|
| 0,65 g | Resorcin |
| 0,50 g | Keratinhydrolysat |
| 0,50 g | Seidenproteinhydrolysat |
| 0,50 g | 2-Amino-6-chlor-4-nitrophenol |
| 0,30 g | Ascorbinsäure |
| 73,75 g | Wasser |
| 100,00 g | |

Die Anwendung erfolgt in der in Beispiel 1 genannten Weise.

Es wird ein gleichmäßiger, kräftiger, leuchtender goldorangefarbener Farbton erhalten, wobei das Haar durch die Färbebehandlung praktisch nicht geschädigt wird.

Alle Prozentangaben stellen, soweit nicht anders angegeben, Gewichtsprozente dar.

**Patentansprüche**

1. Mittel zum Färben von Haaren auf der Basis einer Entwicklersubstanz-Kupplersubstanz-Kombination und gegebenenfalls direktziehenden Farbstoffen, welches vor der Anwendung mit einem Oxidationsmittel vermischt wird und **dadurch gekennzeichnet ist, daß** es eine Kombination aus (a) Bienenwachs und (b) mindestens einem Proteinhydrolysat und/oder mindestens einer Aminosäure enthält.

2. Mittel nach Anspruch 1, **dadurch gekennzeichnet, daß** das Bienenwachs in einer Menge von 0,1 bis 10 Gewichtsprozent enthalten ist.

3. Mittel nach Anspruch 1 oder 2, **dadurch gekennzeichnet, daß** das Proteinhydrolysat ausgewählt ist aus Keratinhydrolysat, Seidenproteinhydrolysat und Mischungen dieser beiden Verbindungen.

4. Mittel nach einem der Ansprüche 1 bis 3, **dadurch gekennzeichnet, daß** die Aminosäure ausgewählt ist aus Glycin, Alanin, Valin, Leucin, Glutaminsäure und Arginin.

5. Mittel nach einem der Ansprüche 1 bis 4, **dadurch gekennzeichnet, daß** es das Proteinhydrolysat und/oder die Aminosäure in einer Menge von 0,1 bis 10 Gewichtsprozent enthält.

6. Mittel nach einem der Patentansprüche 1 bis 5, **dadurch gekennzeichnet, daß** es einen pH-Wert von 7,5 bis 12 aufweist.

7. Mittel nach einem der Ansprüche 1 bis 6, **dadurch gekennzeichnet, daß** es in Form einer Emulsion, eines Geles oder einer Creme vorliegt.

8. Mittel nach einem der Ansprüche 1 bis 7, **dadurch gekennzeichnet, daß** es 0,1 bis 30 Gewichtsprozent eines nichtionischen und/oder anionischen Netzmittels oder Emulgators enthält.

9. Mittel nach Anspruch 8, **dadurch gekennzeichnet, daß** das/der nichtionische und/oder anionische Netzmittel oder Emulgator ausgewählt ist aus Fettalkoholsulfaten, oxethylierten Fettalkoholsulfaten, oxethylierten Fettsäureestern, oxethylierten Nonylphenolen, oxethylierten Fettalkoholen, Alkylbenzolsulfonaten und Fettsäurealkanolamiden.

10. Verfahren zum Färben von Haaren, **dadurch gekennzeichnet, daß** man ein Haarfärbemittel nach einem der Ansprüche 1 bis 9 unmittelbar vor dem Gebrauch mit dem Oxidationsmittel vermischt, eine für die Haarfärbung ausreichende Menge dieser gebrauchsfertigen Färbezubereitung auf das Haar aufträgt und nach einer Einwirkungszeit von 10 bis 45 Minuten bei 15 bis 50 Grad Celsius mit Wasser ausspült und sodann trocknet.

**Claims**

1. Agent for the colouring of hair, based on a developer substance/coupler substance combination and optionally substantive dyes, which is mixed with an oxidizing agent prior to use and which is **characterized in that** it comprises a combination of (a) beeswax and (b) at least one protein hydrolysate and/or at least one amino acid.

2. Agent according to Claim 1, **characterized in that** the beeswax is present in an amount of from 0.1 to 10 percent by weight.

3. Agent according to Claim 1 or 2, **characterized in that** the protein hydrolysate is chosen from keratin hydrolysate, silk protein hydrolysate and mixtures of these two compounds.

4. Agent according to one of Claims 1 to 3, **characterized in that** the amino acid is chosen from glycine, alanine, valine, leucine, glutamic acid and arginine.

5. Agent according to one of Claims 1 to 4, **characterized in that** it comprises the protein hydrolysate and/or the amino acid in an amount of from 0.1 to 10 percent by weight.

6. Agent according to one of Claims 1 to 5, **characterized in that** it has a pH of from 7.5 to 12.

7. Agent according to one of Claims 1 to 6, **characterized in that** it is in the form of an emulsion, a gel or a cream.

8. Agent according to one of Claims 1 to 7, **characterized in that** it comprises 0.1 to 30% by weight of a nonionic and/or anionic wetting agent or emulsifier.

9. Agent according to Claim 8, **characterized in that** the nonionic and/or anionic wetting agent or emulsifier is chosen from fatty alcohol sulphates, oxyethylated fatty alcohol sulphates, oxyethylated fatty acid esters, oxyethylated nonylphenols, oxyethylated fatty alcohols, alkylbenzene-sulphonates and fatty acid alkanolamides.

10. Method for the colouring of hair, **characterized in that** a hair-colouring agent according to one of Claims 1 to 9 is mixed with an oxidizing agent directly prior to use, an amount of this ready-to-use colouring preparation sufficient for the hair colouring is applied to the hair and, after a contact time of from 10 to 45 minutes at 15 to 50° Celsius, the hair is rinsed with water and then dried.

**Revendications**

1. Composition pour la teinture des cheveux, à base d'une association développateur-coupleur et éventuellement de colorants directs, qui est mélangée avant l'emploi avec un oxydant et est **caractérisée en ce qu'**elle contient une association de (a) cire d'abeilles et (b) au moins un hydrolysat de protéine et/ou un aminoacide.

2. Composition selon la revendication 1, **caractérisée en ce que** la cire d'abeilles est contenue en une quantité de 0,1 à 10% en poids.

3. Composition selon la revendication 1 ou 2, **caractérisée en ce que** l'hydrolysat de protéine est choisi parmi l'hydrolysat de kératine, l'hydrolysat de protéine de la soie et des mélanges de ces composés.

4. Composition selon l'une quelconque des revendications 1 à 3, **caractérisée en ce que** l'aminoacide est choisi parmi la glycine, l'alanine, la valine, la leucine, l'acide glutamique et l'arginine.

5. Composition selon l'une quelconque des revendications 1 à 4, **caractérisée en ce qu'**elle contient l'hydrolysat de protéine et/ou l'aminoacide en une quantité de 0,1 à 10% en poids.

6. Composition selon l'une quelconque des revendications 1 à 5, **caractérisée en ce qu'**elle présente un pH de 7,5 à 12.

7. Composition selon l'une quelconque des revendications 1 à 6, **caractérisée en ce qu'**elle se trouve sous forme d'une émulsion, d'un gel ou d'une crème.

8. Composition selon l'une quelconque des revendications 1 à 7, **caractérisée en ce qu'**elle contient de 0,1 à 30% en poids d'un agent mouillant anionique et/ou non ionique ou d'un émulsifiant.

9. Composition selon la revendication 8, **caractérisée en ce que** l'agent mouillant anionique et/ou non ionique ou l'émulsifiant est choisi parmi des sulfates d'alcools gras, des sulfates d'alcools gras oxyéthylés, des esters d'acides gras oxyéthylés, des nonylphénols oxyéthylés, des alcools gras oxyéthylés, des alkylbenzènesulfonates et des alcanolamides d'acides gras.

10. Procédé pour la teinture des cheveux, **caractérisé en ce qu'**on mélange une composition de teinture pour cheveux selon l'une quelconque des revendications 1 à 9, immédiatement avant l'emploi, avec l'oxydant, on applique sur le cheveu une quantité, suffisante pour la teinture des cheveux, de cette composition de teinture prête à l'emploi et, après un temps d'action de 10 à 45 minutes à 15-50°C, on rince à l'eau et ensuite on sèche.